# EUROPEAN PATENT APPLICATION

(11) **EP 1 319 402 A1**
(43) Date of publication of application: **18.06.2003**
(21) Application number: 01955567.1
(22) Date of filing: 03.08.2001
(51) Int. Cl.: A61K 31/202, A61P 43/00, A61P 37/08, A61P 17/00, A61P 11/06, A61P 27/14, A61P 27/16, A61P 1/04

(54) **IGE PRODUCTION INHIBITORS**

(30) Priority: 04.08.2000 JP 2000237679
(71) Applicant: Idemitsu Technofine Co. Ltd, Sumida-ku, Tokyo 130-0015 (JP)
(72) Inventor: NAKAJIMA, Toshiaki c/o Idemitsu Technofine Co. LTD, Tokyo 130-0015 (JP); AOYAMA, Tomoya c/o Idemitsu Technofine Co., LTD., Tokyo 130-0015 (JP); SUZUKI, Osamu, Tsuchiura-shi, Ibaraki 300-0845 (JP); ONO, Kazuhisa c/o Grad. School of Adv. Sci. of Mat, Higashi-hiroshima-shi, Hiroshima 739-852 (JP); KAWAMOTO, Seiji c/o Faculty of Engineering, Higashi-hiroshima-shi, Hiroshima 739-852 (JP)
(74) Representative: Bannerman, David Gardner
(86) International application number: JP0106704
(87) International publication number: WO02011718

(57) **Abstract**

Novel inhibitors of IgE production which comprises at least one or more compound selected from the group consisting of γ-linolenic acid, di-homo-γ-linolenic acid and derivatives thereof as an active ingredient(s) are useful in treating skin conditions and the like in which IgG participate.

## Description

### Technical field

The present invention relates to inhibitors of IgE production, preferably drugs available in treating diseases caused by IgE production, particularly in treating skin symptoms.

### Background art

γ-Linolenic acid, which is one of typical unsaturated fatty acids, is a linear trienoic fatty acid having *cis* double bonds at positions 6, 9, and 12 and contains 18 carbon atoms. γ-Linolenic acid is contained in evening primrose seed oil in 8 to 10% and is used in health-care foods and the like.

γ-Linolenic acid is also used as a component of various pharmaceuticals. For example, therapeutic agents for allergic rhinitis and allergic asthma containing γ-linolenic acid as an active ingredient (JP 61-87621 A), medical compositions containing γ-linolenic acid or di-homo-γ-linolenic acid or the like for treating skin pruritus and hyperparathyroidism of patients requiring artificial dialysis, (JP 7-233062 A), fortified fruit juice containing γ-linolenic acid or di-homo-γ-linolenic acid effective for treating or preventing atopic eczema, rheumatoid arthritis, coronary vascular diseases, asthma, diabetes, prostatic disorders, or the like (JP 8-205832 A), external preparation for skin treatment after depilation containing γ-linolenic acid or di-homo-γ-linolenic acid (JP 10-218731 A), or the like are known.

However, the drugs containing γ-linolenic acid as described above have been explained to be useful due to their anti-inflammatory actions, and it has been unknown that γ-linolenic acid has an activity to inhibit IgE (immunoglobulin E) production.

### Disclosure of the Invention

An object of the present invention is to provide a novel inhibitor of IgE production which can be used in treating diseases caused by IgE production, particularly in treating skin conditions or the like.

To attain the above-mentioned object, the inventors of the present invention have made extensive studies and as a result, they have found that γ-linolenic acid has an activity to inhibit IgE production, thereby achieving the present invention.

Namely, the present invention relates to an inhibitor of IgE production comprising one or more compound selected from the group consisting of γ-linolenic acid, di-homo-γ-linolenic acid and derivatives thereof as an active ingredient.

Further, the present invention relates to an inhibitor of IgE production, wherein the inhibitor is to be administered at 0.3 mg/kg/day or more as an amount of the active ingredient.

Hereinafter, the present invention will be described in detail.

The inhibitor of IgE production of the present invention contains one or more compound selected from γ-linolenic acid, di-homo-γ-linolenic acid, and derivatives thereof (hereinafter, also referred to as "γ-linolenic acid, etc.") as active ingredients.

γ-Linolenic acid, etc. have a limited natural source of supply. Usually, they are available from oils and fats contained in fungi such as the genera *Nortierella, Mucor, Rhizopus*, etc.; plants such as evening primrose and borage; algae such as *Spirulina*, etc. The purified products as well as the extracts from these may be used. Furthermore, fungi or *Spirulina*, etc. themselves may be used as they are without extraction. Alternatively, γ-linolenic acid can be obtained by chemical synthesis or commercially available products may be used. Furthermore, extracts and semi-purified products from microorganisms or plants which contain γ-linolenic acid or its derivatives can also be used as long as they are pharmaceutically acceptable.

Derivatives of γ-linolenic acid, etc. include esters obtained by the reaction between γ-linolenic acid and various alcohols, for example, ethyl ester, glycerol ester, phospholipids, etc., or salts obtained by reacting γ-linolenic acid, etc. with inorganic or organic bases in equimolar ratios, for example, sodium salts, potassium salts, etc.

γ-linolenic acid, etc. are essential fatty acids used in foods, so that there will be particularly anticipated no problem in safety.

The inhibitor of IgE production of the present invention can contain, besides γ-linolenic acid, etc. as active ingredients, those components which are usually employed in pharmaceuticals or health-care foods, etc.

The form of the inhibitor of IgE production of the present invention is not particularly limited. Single compound or mixture of two or more compounds selected from γ-linolenic acid, di-homo-γ-linolenic acid, and derivatives thereof, extracts from oils and fats of the above-mentioned fungi and plants, or the microorganism themselves, etc. can be mixed with one or more kinds of vehicles, carriers, excipients, integrators, preservatives, stabilizers, flavors, etc. that are pharmaceutically acceptable and harmless in general to obtain preparations for internal use such as tablets, granules, capsules and solutions; suppositories; external vaginal preparations; preparations for external use such as ointments, creams and lotions; injections such as sterilized solutions and suspensions. These preparations can be produced by conventional techniques.

For example, the above-mentioned γ-linolenic acid, etc., may be mixed with a binder such as cornstarch or gelatin, an excipient such as crystalline cellulose, a swelling agent such as potato starch or sodium alginate, a sweetener such as lactose or sucrose, etc. to form powder, tablets, pills or granules. Capsules can be prepared by filling a mixture of γ-linolenic acid, etc. and other oils and fats in soft gelatin capsules, hard gelatin capsules or the like in a conventional manner. Furthermore, cyclodextrin inclusion compounds may be prepared from cyclodextrin and γ-linolenic acid, etc. in a conventional manner. In the case of preparations for external use, vaseline, paraffin, oils and fats, lanolin, etc. are used as bases.

To the above-mentioned γ-linolenic acid, etc., ω3-series unsaturated fatty acids such as α-linolenic acid, eicosapentaenoic acid and docosahexaenoic acid; ω5-series unsaturated fatty acids such as myristoleic acid, ω7-series unsaturated fatty acids such as palmitoleic acid, ω9-series unsaturated fatty acids such as oleic acid and erucic acid, saturated fatty acids such as lauric acid and myristic acid may be added in any ratio. To prevent oxidation of γ-linolenic acid or other fatty acids, antioxidants such as vitamin E, ascorbyl palmitate and ascorbyl stearate may be added.

In addition to γ-linolenic acid, etc., other medicinal ingredients having an IgE-inhibitory activity may be used in combination.

The mixing ratio of γ-linolenic acid, etc. to total amount of the inhibitor of IgE production is preferably 0.000001 to 100% by weight, more preferably 0.000018 to 100% by weight against the whole.

It has been revealed that administration of γ-linolenic acid, etc. before the induction of IgE production or at an initial stage thereof results in higher inhibition of IgE production. Therefore, the inhibitor of IgE production of the present invention can effectively inhibit the production of IgE which causes immediate allergy, so that it can prevent symptoms of any disease caused by IgE production. That is, it can be used in preventive treatment. The diseases caused by IgE production include skin diseases caused by IgE production, atopic dermatitis, asthma, allergic rhinitis, allergic enteritis, pollinosis, allergic conjunctivitis, etc.

From the above, the inhibitor of IgE production of the present invention can be administered to healthy persons and persons who have a constitution with a tendency to suffer from the above-mentioned diseases in order to prevent the occurrence of the above-mentioned diseases. In particular, it is effective to treat patients who are expected to suffer from the diseases to prevent the onset thereof. In the case where IgE production has not increased, it is expected that administration of the inhibitor of IgE production to patients who are in initial stages of the above-mentioned diseases can alleviate the symptoms.

The dosage is not particularly limited so long as the amount of γ-linolenic acid, etc. is sufficient for effective prevention of IgE production. However, if the dosage becomes too much, soft stool tends to occur. The dosage can be set appropriately depending on the age, body weight, and medical history of the patient , the kind and the symptom, etc. of the disease. In preventive treatment of diseases caused by IgE production, a desired effect can be expected by the administration of γ-linolenic acid in a daily dosage of preferably 0.3 to 1,000 mg/ kg body weight of the administered person, more preferably 1 to 500 mg/kg.

### Brief Description Of the Drawings

Fig. 1 is a graph showing a change with time of disease score of NC/Nga mice;
Fig. 2 is a graph showing a change with time of total IgE amount in plasma of NC/Nga mice;
Figs. 3 are graphs showing a change with time of disease score of NC/Nga mice;
Fig. 4 is a graph showing a change with time of total IgE amount in plasma of NC/Nga mice;
Fig. 5 is a graph showing IgE amount in culture supernatant at each concentration of added GLA;
Fig. 6 is a graph showing cell proliferation ability (absorbance at 405 nm) at each concentration of added GLA.

### Best Mode For Carrying Out the Invention

First, the model animal used in the examples will be explained.

### [Model Animal]

As a model animal, NC/Nga mouse was used. The NC/Nga mouse is an inbred mouse established in 1957, which is originated from "Nishiki" mouse, a pet mouse. Long since, it has been known that this mouse is sensitive to X-rays and to ovalbumin-caused anaphylactic shocks. In 1997, Matsuda et al. revealed that the mouse shows a phenotype similar to atopic dermatitis (hereinafter, referred to as "AD") in humans (Int. Immunol., 9, 461-466, 1997). That is, it was revealed that the mouse presents AD-like clinical features with severe pruritus, inflammation, bleeding, edema and dry skin and that it suffers from hyperIgE syndrome. Histopathological finding on its skin indicated that infiltration of inflammatory cells such as AD-like mast cells and eosinophilic leucocytes, etc. and hyperplasia of skin tissue were observed. Such AD-like phenotype occurs in the ordinary environment but not in a SPF (specific pathogen-free) environment where neither microorganism nor parasite exists, so that the onset of the disease can be controlled. In this view, the mouse was used as a disease model of atopic dermatitis in carrying out the present invention

Until now, there has been reported no experimental results on the study of whether or not high IgE production is necessary for causing AD-like diseases in NC/Nga mouse. While, the inventors of the present invention compared the IgE amount between a group in which the disease was considerably aggravated and a group in which the disease was not so aggravated, in consideration of individual differences in NC/Nga mice. As a result, the inventors of the present invention confirmed that the IgE level of the former group was significantly high, which suggests that the aggravation of the disease and an increase in IgE production correlate to each other as far as the previous reports are referred.

### [Example 1]

It has long since been suggested clinically that oral administration of evening primrose oil which is rich in γ-linolenic acid is effective in treating AD. On the contrary, some clinical results showing ineffective have also been obtained. In consideration of problems such as differences in the condition of each clinical trial and the purity of evening primrose oils, it has been considered that the true effect of γ-linolenic acid (hereinafter, referred to as "GLA") cannot be evaluated exactly so far. In this example, the effect of oral administration of purified GLA in the NC/Nga mice, an AD model, was studied.

As the model animal, 5-week-old male NC/Nga mice bred in SPF environment, presenting neither dermatitis-like feature nor hyperIgE syndrome were used. To these mice, GLA ethyl ester (degree of purification of 95.98%: manufactured by Idemitsu Material Co., Ltd.) was administered by a stomach tube method in a dosage of 50 mg/individual once every other day starting from 5-week-old to obtain a GLA-administered group composed of 6 animals. On the other hand, as a control, PBS (phosphate buffered saline) instead of GLA ethyl ester was likewise administered in a dosage of 50 µl/individual to obtain a control group composed of 6 animals.

In each week from the onset of administration (5 weeks old) up to 19 weeks old, the disease scores of the above-mentioned mice were converted into points according to the method of Matsuda et al. (Int. Immunol., 9, 461-466, 1997). A concrete method is described below.

The following 5 items from A to E were scored by a three point full mark scoring system of 0 (none), 1 (slightly aggravated), 2 (moderately aggravated), and 3 (considerably aggravated). In total, 15 points make a full score.
A (pruritus): Each mouse was observed for 3 minutes to score the frequency at which it scratches.
B (erythema, bleeding): Bleeding from ears and face and erythema on the back of ears were scored.
C (edema): Mainly, degree of swelling of ears was scored.
D (abrasions, erosion): Abrasions around the face, on the back of ears, and at around the base of arm were scored.
E (dryness): Degree of dry skin from the back to the face was scored.

In addition, blood was collected from the above-mentioned mice at the time of starting the administration (5 weeks old), and at 7, 9, and 11 weeks old and was centrifuged at 1,500 rpm for 10 minutes to obtain plasma. Then, total IgE amount in the plasma was measured by a sandwich ELISA method. A concrete method thereof is described below.

Rat anti-murine IgE monoclonal antibody (manufactured by PharMingen Co.) diluted to 2 µl/ml with coating buffer (0.1 M NaHCO₃, 0.5 M NaCl, pH 8.5) was added to each well of a plate (NUNC-IMMUNOPLATE, manufactured by NUNC Inc., A/S) in an amount of 50 µl, and left to stand at 4°C overnight or at 37°C for 3 hours, followed by washing the wells with PBS/Tween (PBS, 0.05% Tween-20, pH 7.5) 3 times.

To each well, 200 µl of blocking buffer (PBS containing 2% skimmed milk) was added and incubated at 37°C for 3 hours, followed by washing the wells with PBS/Tween 3 times.

Then, the above-mentioned murine plasma was diluted 100 folds with dilution buffer (PBS, 2% skimmed milk, 0.25% SDS) to prepare a sample for measurement. Furthermore, murine IgE standard (manufactured by PharmMingen Co.) was diluted with dilution buffer to prepare a standard for measurement.

The samples for measurement and standard were added to each well in amounts of 50 µl/well and incubated at 37°C for 3 hours, followed by washing the wells with PBS/Tween 3 times.

Biotinylated rat anti-murine IgE antibody (manufactured by PharmMingen Co.) diluted to 2·µg/ml with dilution buffer was added to each well in an amount of 50 µl and incubated at 37°C for about 3 hours, followed by washing the wells with PBS/Tween 6 times.

Furthermore, alkaline phosphatase-labeled Streptoavidin (manufactured by BioSource, Inc.) diluted to 1,000 folds with dilution buffer was added to the wells in an amount of 50 µl/well and incubated at 37°C for 1 hour, followed by washing the wells with PBS/Tween 6 times.

Then, AttoPhos™ (manufactured by BOEHRINGER MANNHEIM GmBH) was added to each well in an amount of 50 µl and left to stand until coloring occurred in a light-shielded state (for about 6 hours), and the fluorescence intensity was measured by using CytoFluor™ II (PE Biosystems, Co.), from which total IgE amount was calculated.

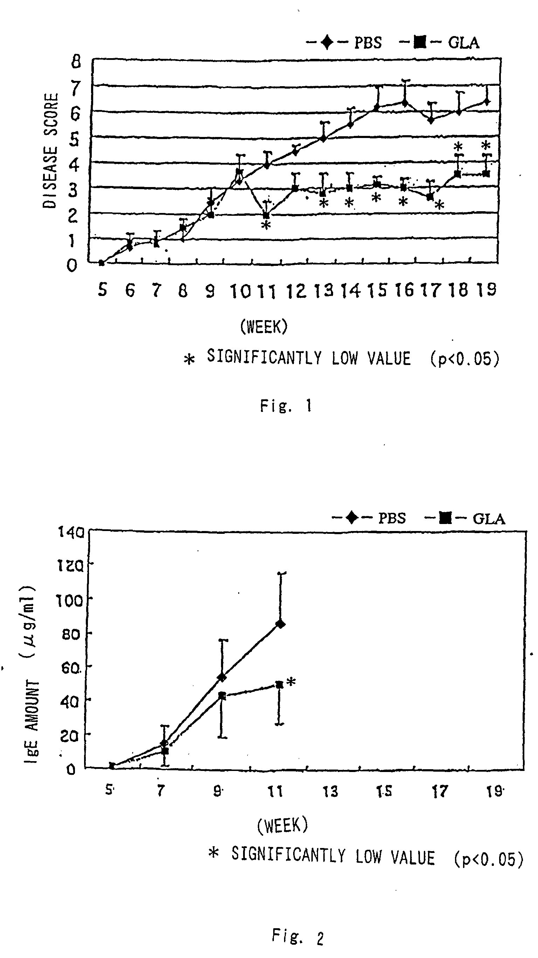

The results on the disease scores of mice are shown in Fig. 1.

In the control group (PBS-administered group), the disease score significantly increased with time. In contrast, in the GLA-administered group, no significant increase was observed at week 11 or thereafter. Comparison with the control group revealed that the GLA-administered group showed significant inhibition of the disease score at week 11 and weeks 13 to 19.

Also, the results of total IgE amount in murine plasma are shown in Fig. 2.

In both the control group and the GLA-administered group, there was observed a tendency that total IgE amounts increase with a lapse of time. However, it was revealed that the GLA-administered group was low in total IgE amount at each measurement period as compared with the control group and in particular at week 11, the total IgE amount was significantly low.

From the above, it is evident that GLA has an activity to inhibit IgE production.

The above-mentioned results indicate that oral administration of GLA inhibits AD-like disease and high IgE production in NC/Nga mice. The results in this example were the first to exhibit the anti-atopic effect and IgE production inhibitory effect of GLA in an animal disease model. These strongly suggest usefulness of GLA in designing functional foods having AD preventive effects for next generation.

### [Example 2]

The tests of oral administration of 50 mg GLA revealed the effect of GLA to inhibit progress of disease and high IgE production in NC/Nga mice. Therefore, studies were made on the effects of oral administration of GLA to the mice in different dosages and administration form.

As the model animal, 5-week-old male NC/Nga mice bred in SPF environment, presenting neither dermatitis-like feature nor hyperIgE syndrome were used. The animals were divided into each group composed of 10 animals. For the GLA-administered group, GLA ethyl ester (degree of purification of 95.98%: manufactured by Idemitsu Material Co., Ltd.) was administered by a stomach tube method in a dosage of 0.1 mg/individual once every other day. Administration of GLA was performed by using, olive oil containing little essential fatty acid which was added to 0.1 mg of GLA ethyl ester to make 50 µl. For the control group, 50 µl of olive oil alone was likewise administered.

The disease score of mice in these groups were calculated every week in the same manner as in Example 1 and total IgE amount in plasma was measured once every other week.

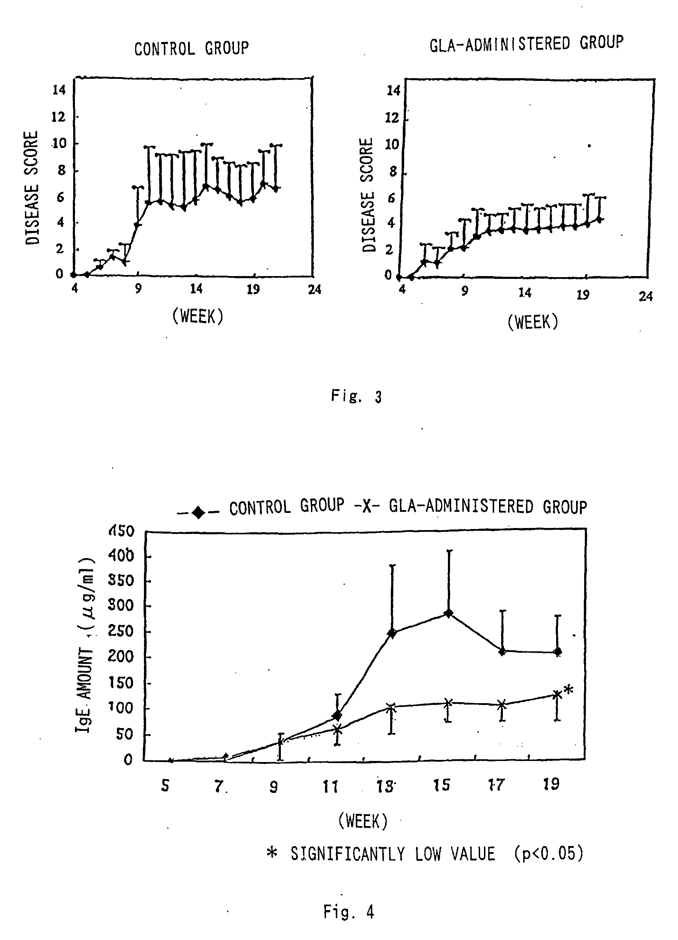

The results of the disease score of these mice are shown in Figs. 3.

Although some deviation was observed in the score due to individual differences in mice, totally, an increase in the score was observed from week 6. In the control group, dry skin occurred at week 8 and the number of scratching increased, resulting in bleeding around the eye, in the ears, and on the face. Also, on the body, erythema started to be observed. As a result, an abrupt increase in the score was observed and the condition was always bad and no improvement was observed despite some fluctuations. On the contrary, the GLA-administered group showed low average scores at week 9 or thereafter as compared with the control group. In particular, at weeks 15 to 17, they showed significantly low scores.

The results of total IgE amount in murine plasma are shown in Fig. 4.

From week 7 to week 9, total IgE amount started to increase, reaching a peak at week 17 in the control group (see the curve indicated by the symbol ◆ in Fig. 4). In contrast, in the GLA-administered group, an increase was observed at and after week 9 but no significant increase was observed at week 13 and thereafter, this group showed significantly low values as compared with the control group up to week 13 to week 19 (see the curve indicated by the symbol × in Fig. 4).

The study of the effect of oral administration of olive oil containing 0.1 mg GLA ethyl ester in this example indicated that both the IgE amount and the disease score were significantly low.

### [Example 3]

Examples 1 and 2 described above demonstrated that oral administration of GLA has the effect of inhibiting both the progress of AD-like disease and high IgE production in NC/Nga mice. In this example, the effect of GLA to inhibit IgE production *in vitro* was studied by an *in vitro* class switch induction system using spleen cells of the mice.

First, spleen cells were collected from NC/Nga mice by the following method.

An 8-week-old NC/Nga mouse bred in SPF environment, presenting neither dermatitis-like feature nor hyperIgE syndrome was sacrificed by dislocation fracture of the cervical vertebrae and the spleen was extracted in a sterile condition. The extracted spleen was placed in RPMI-1640 medium (about 3 ml) in a dish, and separated into individual cells with a sterilized pincette. The separated cells were placed in a 15-ml Falcon tube and the membrane of the spleen was removed. Then, the cells suspended in the medium were centrifuged at 1,500 rpm for 5 minutes to recover only the cells. Subsequently, to remove erythrocytes, the recovered cells were suspended in 10 ml of sterilized lysis buffer at 4°C to cause hemolysis, followed by washing twice with sterilized PBS produced by Milli-Q at 4°C to collect spleen cells. A portion of the obtained spleen cells was stained with Trypan Blue (Trypan Blue Stain 0.4%: manufactured by LIFE TECHNOLOGIES, INC.) and counted on a hemacytometer.

Then, measurement of IgE amount in the culture supernatant in a class switch induction system was performed by the following procedure.

Based on the number of cells counted by the above-mentioned method, the cells were inoculated on a plate (96-well cell culture microtest plate, with flat bottom, low evaporation type, made of polystyrene with a lid: manufactured by FALCON CO.) to a concentration of 2 × 10⁵ cells/ml per well.

The medium used here contained cytokine IL-4 (100 U/ml) and LPS (10 µg/ml) as stimulants for the IgE class switch induction.

To observe the influences of GLA before, during, or after the stimulation for class switch, GLA was added to the media in various concentrations (0, 10, 20, 40, 60, 80, 120, 160 µg/ml) after 0 hour (at the start of culture), 48 hours, or 72 hours from the start. Here, the addition of GLA was performed by diluting it with ethanol to appropriate concentrations and making the final concentration of ethanol to be 0.1%.

All the cultures were performed at 37°C in a 5%-CO₂ incubator and the cultured supernatants were recovered after 7 days (after 168 hours). The IgE amounts thereof were measured by a sandwich ELISA method.

Furthermore, to study the influence of added GLA on the proliferation of cells, the cell proliferation potency of cells was measured by the following method. Based on the number of cells counted by the above-mentioned method, 2 × 10⁵ cells/ml of the cells in RPMI-1640 medium was inoculated in each well and IL-4 (100 U/ml) and LPS (10 µg/ml) were added as stimulants.

After 0 hour of culture (at the time of starting the culture), GLA was added to the media in various concentrations (0, 10, 20, 40, 60, 80, 120, and 160 µg/ml). Here, the addition of GLA was performed by diluting it with ethanol to appropriate concentrations and making the final concentration of ethanol to be 0.1%. As controls, media with only IL-4or LPS as the stimulant for the class switch and media without stimulant were prepared.

About the cell proliferation potency of the cells in the above-mentioned media, the proliferation activity during the period between 60 and 72 hours from the start of the culture was measured. After 60 hours from the start, the BrdU labeling solution contained in a BrdU Labeling & Detection Kit (manufactured by BOEHINGER MANNHEIM GmBH) was added to the media in a ratio of 20 µl/200 µl of medium so as to obtain a final concentration of 110 µM, followed by culturing the cells in an ordinary manner at 37°C in a 5%-CO₂ incubator up to 72 hours.

After 72 hour of culture, the cells were centrifuged at 300 rpm for 10 minutes and then, which was kept at 60°C in an oven with the lid of the culture plate left open in order to dry up the media. After about 6 hours of drying, a fixing solution (70% ethanol, 0.5 M hydrochloric acid) cooled to -20°C was added to the wells in an amount of 200 µl/well and the cells were fixed at -20°C for 30 minutes. Excess BrdU and fixing solution were completely removed by washing with PBS three times and 100 µl of a nuclease solution was added thereto, followed by performing reaction at 37°C for 30 minutes.

Again, washing with PBS was performed three times and 100 µl of a solution of anti-BrdU-POD antibody containing 10 mg/ml BSA was added and left stand at 37°C for 30 minutes. Then, after washing three times with the washing buffer attached to the kit, coloring was performed with a peroxidase substrate solution. After 10 minutes from the addition of the substrate, absorbance at 405 nm was measured by using a plate reader.

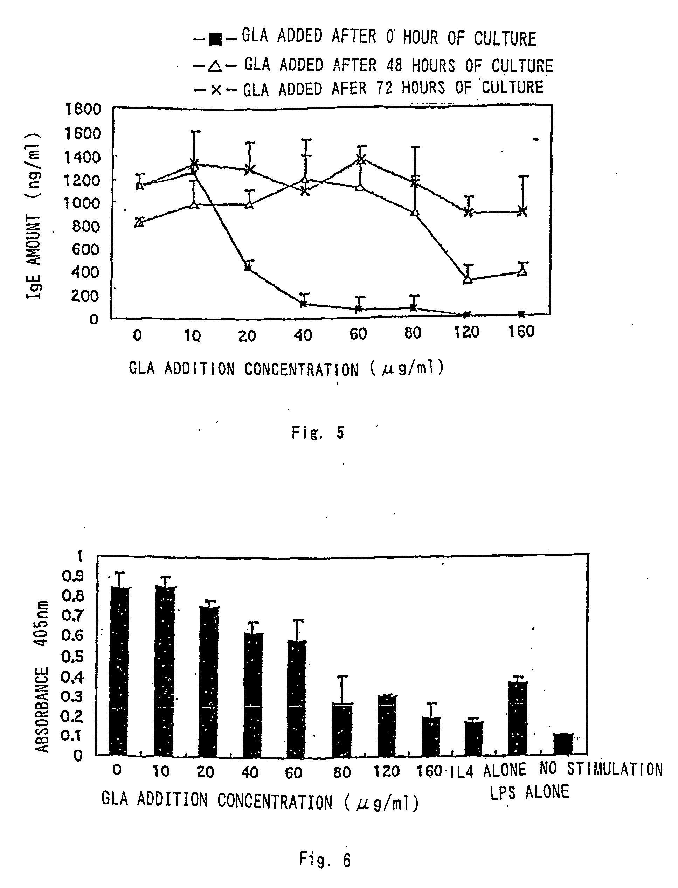

Fig. 5 shows the results of measurements of IgE amounts in the culture supernatants when GLA was added in the class switch induction system.

In the case where the addition was made after 0 hour of culture, total amount of produced IgE decreased almost together with an increase of added-GLA concentration, indicating that GLA inhibits IgE production dose-dependently, with complete inhibition of the IgE production at the concentration of 120 µg/ml of added GLA (see the curve indicated by symbol in Fig. 5).

In the case where the addition was made after 48 hours of culture, no IgE inhibition was observed up to the concentration of 80 µg/ml, while above the concentration of 120 µg/ml, not complete but significant inhibition occurred (see the curve indicated by symbol in Fig. 5).

In the case where the addition was made after 72 hours of culture, no significant inhibition of IgE production was observed at the added concentrations measured (see the curve indicated by symbol in Fig. 5).

Above results show that GLA has the ability to inhibit IgE production *in vitro,* too, and that, besides, it inhibits IgE production dose-dependently when it is made to act in an initial stage of IgE production including a class switch.

However, only from the above-mentioned results, it may be considered that the decrease in the produced IgE amount could be caused by certain GLA-mediated control of the class switch itself, or by the reduction in the number of IgE-producing cells as a whole caused by GLA-mediated control of cell proliferation.

Then, the cell proliferation potency at the time of GLA addition was measured. The results obtained are shown in Fig. 6.

As a result, a decrease in the cell proliferation potency was observed according to an increase of added GLA concentration; however, at each added GLA concentration, the decrease in cell proliferation did not occur in the same ratio as that of the decrease in the produced IgE amount. In particular, at the concentration of added GLA within the range of 20 to 60 µg/ml, degree of the decrease in the total IgE amount produced was revealed to be significantly greater than that of the decrease in cell proliferation (the curves indicated by symbol in Figs. 6 and 5). Concretely, µcompared to no-GLA-added (0 µg/ml) lot, 40-µg/ml-GLA-added lot was revealed to show about one tenth decreased amount of totally produced IgE, while its cell proliferation did not substantially decrease.

The above results suggest the possibility that the inhibition of IgE production by GLA is not due to the GLA-mediated inhibition of cell proliferation but that GLA inhibits the IgE production in another route.

The experiments in this example have made it evident that GLA exhibits the effect of inhibiting IgE production *in vitro,* too. In the present experimental system, GLA showed no inhibitory effect when added 72 hours after the stimulation with cytokine(s) which induce IgE production, thus, it is presumed that GLA inhibits the IgE production in its initial stage including class switch recombination of antibody gene. In addition, the results seem to be consistent with the *in vivo* results where 2 month-long oral administration of GLA to AD-suffering NC/Nga mice with hyperIgE syndrome showed no inhibition of IgE production. Since the inhibitory effect of GLA on IgE production is not due to cytotoxicity or cell proliferation inhibition, it may be caused as a result of inhibition of a certain stage in the IgE production mechanism (for example, embryo type transcription in the IgE constant region, cleavage and repair of double strand DNA in the class switch recombination, differentiation of IgE positive B cells into antibody producing cells, etc.).

### Industrial Applicability

The inhibitor of IgE production of the present invention has excellent inhibitory effect on IgE production, so that it is useful for diseases caused by IgE production.

## Claims

1. An inhibitor of IgE production comprising at least one or more compound selected from the group consisting of γ-linolenic acid, di-homo-γ-linolenic acid and derivatives thereof as an active ingredient.

2. An inhibitor of IgE production according to claim 1, wherein the inhibitor is to be administered at 0.3 mg/kg/day or more as an mount of the active ingredient.
